# EUROPEAN PATENT APPLICATION

(11) **EP 3 254 704 A1**
(43) Date of publication of application: **13.12.2017**
(21) Application number: 16746792.7
(22) Date of filing: 28.01.2016
(51) Int. Cl.: A61L 2/18

(54) **HYDROGEN PEROXIDE SOLUTION SUPPLY APPARATUS**

(30) Priority: 03.02.2015 KR 20150016647
(71) Applicant: CMTECH CO., LTD., Daegu 41059 (KR)
(72) Inventor: MIN, Heung Sik, Daegu 41059 (KR); AHN, Young Keun, Daegu 42605 (KR); YANG, Sung Jin, Daegu 42618 (KR); MIN, Jin Woo, Daegu 41107 (KR)
(74) Representative: Zeitler Volpert Kandlbinder Patent- und Rechtsanwälte Partnerschaft mbB
(86) International application number: PCT/KR2016/000946
(87) International publication number: WO 2016/126051

(57) **Abstract**

The present invention relates to a sterilizer using a hydrogen peroxide solution as a disinfectant, which includes: a disinfectant lifting and lowering unit capable of housing and transporting a disinfectant container filled with disinfectant; a sting unit which opens the disinfectant container; and a drain unit which drains discharging disinfectant from the disinfectant container, wherein the disinfectant lifting and lowering unit and the stinger unit are lifted and lowered along a transport guide provided in the housing. Since it is possible to supply hydrogen peroxide to the sterilizer without frequent replacement of the hydrogen peroxide container as disinfectant many times, there is an effect that sterilization of the sterilizer can be quickly performed.

## Description

### TECHNICAL FIELD

The present invention relates to a hydrogen peroxide supply apparatus, and more particularly, to a hydrogen peroxide supply apparatus in which a given quantity of hydrogen peroxide solution is supplied to a sterilizer even without frequent exchange of a hydrogen peroxide solution container when supplying a hydrogen peroxide solution as disinfectant to a sterilizer, thereby providing a precise sterilization.

### BACKGROUND

Medical devices are usually sterilized, using a high-pressure steam sterilization method which utilizes water vapour saturated under high pressure, or using an ethylene oxide gas sterilization method which utilizes a chemical substance such as ethylene oxide which does not damage the heat sensitive mechanism or material.

However, since the high-pressure steam sterilizer performs sterilization at a high temperature of 120 degrees or higher, medical devices made of synthetic resin which have been developed recently will be deformed, and lifetime of the medical devices made of steel will be further reduced than a usual lifetime due to the fact that the sharp blades become blunt. In particular, since expensive medical devices, tools and apparatuses which are increasing with the development of the latest surgical techniques are sensitive to heat or moisture and may be damaged during sterilization and reprocessing process, the high-pressure steam sterilization method may be an unsuitable sterilization method.

Ethylene oxide gas sterilizers capable of minimizing damage to the device can be sterilized at low temperature. However, because ethylene oxide remains in an object to be sterilized, or because carcinogenesis and toxicity substances can be produced due to the reaction product thereof, a ventilation time of about 12 hours or more after sterilization is required. In addition, there is a report that ethylene oxide gas itself has a high risk of explosion and may act as a genetic toxic substance which may cause a mutation, and ethylene oxide gas is defined as a carcinogenic substance. Thus, its use requires much attention.

Meanwhile, the sterilization method using hydrogen peroxide vapour has a short sterilization time of 30 to 60 minutes at a temperature of 40 to 50 degrees, and a substance which is released into the atmosphere after sterilization to be harmless to the human body or the environment is water and oxygen. Thus, the sterilization method can complement the drawbacks of high-pressure steam sterilizer and various drawbacks of ethylene oxide gas sterilizer.

For an effective sterilization, it is desirable to use a more concentrated hydrogen peroxide solution, but when the concentration of the hydrogen peroxide solution is 60% by weight or more, it is practically difficult to perform treatment such as transportation and storage.

As one of methods for solving these drawbacks, "Hydrogen Peroxide Plasma Sterilization System" of Korean Patent No. 0132233 discloses a technique for bringing a sterilized article to be sterilized into contact with hydrogen peroxide vapour in advance, and then generating actives pieces from hydrogen peroxide to perform sterilization, and decomposing and removing hydrogen peroxide remaining in the object to be sterilized into non-toxic substances, using plasma.

In the low-temperature plasma sterilization and disinfection system, a device for supplying hydrogen peroxide adopts a capsule type cassette way in which a certain amount of hydrogen peroxide solution is injected, and when the cassette is moved to an injector valve assembly, the solution contained in the capsule is moved to a vaporizer due to a pressure difference caused by a sterilization chamber vacuum, is vaporized into gas and is supplied into the sterilization and disinfection reactor.

However, such a method has a drawback in which, after ten sterilization processes are completed with a group of ten capsule cassettes in which one capsule is used in one sterilization and disinfection process, there is a need to replace the old cassette with a new cassette to which ten pieces are injected again, and such a device has a drawback in which the structure of a device which quantitatively supplies a small amount of liquid is very complicated and the cost of the device is high.

In order to solve the aforementioned problems, there is a need for a hydrogen peroxide supply apparatus in which the inconvenience caused by the frequent exchange of the cassettes containing hydrogen peroxide as disinfectant is eliminated, the manufacturing cost is low due to a simple structure, and a small amount of hydrogen peroxide solution can be automatically and quantitatively supplied.

### SUMMARY

The present invention is devised to solve the above-mentioned problems, and an object of the present invention is to provide a hydrogen peroxide supply apparatus capable of supplying hydrogen peroxide many times, without frequent exchange of a hydrogen peroxide container as a disinfectant.

Another object of the present invention is to provide a hydrogen peroxide supply apparatus which quantitatively supplies a liquid of a small amount of hydrogen peroxide solution to a sterilizer, while having a simple apparatus structure.

The objects of the present invention are not limited to the above-mentioned objects, and another object which has not been mentioned may be clearly understood by those skilled in the art from the description below.

In order to solve the problems pointed out above, the present invention provides a hydrogen peroxide supply device including: a disinfectant lifting and lowering unit which transports a disinfectant container; a stinger unit which opens the disinfectant container; a drain unit which drains disinfectant from the disinfectant container; and a transport guide which transports the disinfectant lifting and lowering unit and the stinger unit.

Further, the present invention provides the hydrogen peroxide supply device, wherein the stinger unit further including: a transport unit which moves up and down along the transport guide; a stinger fixed to the bottom of the transport unit to open the disinfectant container; a fixing unit to which a lid of the disinfectant container is inserted and fixed; and an elastic unit which connects the transport and the fixing unit, wherein the fixing unit further includes a through-hole through which the stinger is guided.

Further, the present invention provides the hydrogen peroxide supply device, wherein the stinger includes: a tip portion which opens the lid of the disinfectant container; and a tubular body portion.

Further, the present invention provides the hydrogen peroxide supply device, wherein the drain unit includes: a drain pipe which drains the disinfectant; and support means which fixes the drain pipe to the housing.

Further, the present invention provides the hydrogen peroxide supply device, wherein the drain pipe is provided in a state in which a part thereof is inserted into an inner diameter of the stinger body portion, and the inner diameter of the stinger body portion is formed to be larger than an outer diameter of the drain pipe.

Further, the present invention provides the hydrogen peroxide supply device, wherein the drain pipe includes: a first drain pipe which supplies the disinfectant drained from the disinfectant container to the sterilizer; and a second drain pipe which is diverged from a point of the first drain pipe at a position close to the sterilizer to re-discharge or circulate the residue inside the first drain pipe to the disinfectant container.

In the hydrogen peroxide supply apparatus according to the present invention as described above, since hydrogen peroxide can be supplied many times to the sterilizer of the container of hydrogen peroxide solution as the disinfectant, there is an effect in which the sterilization of the sterilizer can be quickly performed.

In addition, the present invention has an effect of quantitatively supplying a small amount of hydrogen peroxide solution liquid to the sterilizer, while having a simple apparatus structure.

### BRIEF DESCRIPTION OF THE DRAWINGS

FIG. 1 is a schematic perspective view illustrating a configuration of a sterilizer to which a hydrogen peroxide supply apparatus according to the present invention is applied;
FIG. 2 is a perspective view illustrating a hydrogen peroxide bottle mounted in the hydrogen peroxide supply apparatus of the present invention;
FIG. 3 is a perspective view illustrating the hydrogen peroxide supply apparatus of the present invention;
FIG. 4 is a cross-sectional view of FIG. 3;
FIG. 5 is a view illustrating a state in which the hydrogen peroxide supply apparatus of the present invention is connected to the vaporizer of the sterilizer; and
FIGS. 6 to 10 are schematic cross-sectional views illustrating the operation procedure of the hydrogen peroxide supply apparatus according to the present invention.

### DETAILED DESCRIPTION OF THE INVENTION

Advantages and features of the present invention and methods of achieving the same will become apparent with reference to the embodiments described in detail below in conjunction with the accompanying drawings. However, the present invention is not limited to the embodiments disclosed below, but may be provided in various different forms. The present embodiments are merely provided to make the disclosure of the present invention complete and to fully inform the category of the invention to a person having ordinary knowledge in the technical field to which the present invention pertains, and the present invention is only defined by the scope of the claims.

Specific contents for carrying out the present invention will be described in detail with reference to the accompanying drawings. Regardless of the drawings, the same reference numerals refer to the same elements, and the term "and/or" includes each of the mentioned items and one or more combinations.

Although the terms "first, second, and the like" are used to describe various constituent elements, these constituent elements are of course not limited by these terms. These terms are merely used to distinguish one constituent element from other constituent elements. Therefore, it is a matter of course that the first constituent element described below may be a second constituent element within the technical idea of the present invention.

The terms used in the present specification are for the purpose of illustrating the examples and do not limit the present invention. As used herein, the singular form also includes the plural forms unless specifically stated in a phrase. The terns "comprises" and/or "comprising" used in the specification do not exclude the presence or addition of one or more other constituent elements in addition to the referenced constituent elements.

Unless otherwise defined, all terms (including technical and scientific terms) used in the specification can be used in the meaning capable of being commonly understood by those having ordinary skill in the technical field to which the present invention pertains. Also, commonly used predefined terms are not ideally or unduly interpreted unless otherwise clearly especially defined.

Spatially relative terms "below", "beneath", "lower", "above", "upper" and the like may be used to easily describe the correlation between one constituent element and another constituent element as illustrated in the drawing. Spatially relative terms should be understood as terms including different directions of the constituent elements during use or operation in addition to the direction illustrated in the drawings. For example, when reversing a constituent element illustrated in the drawings, a constituent element described as "below" or "beneath" of another constituent element may be located "above" another constituent element. Thus, the exemplary term "below" may include both of the lower and upper directions. The constituent elements may also be oriented in other directions, and the spatially relative terms can be interpreted by orientation accordingly.

Hereinafter, preferred embodiments of the present invention will now be described in detail with reference to the accompanying drawings.

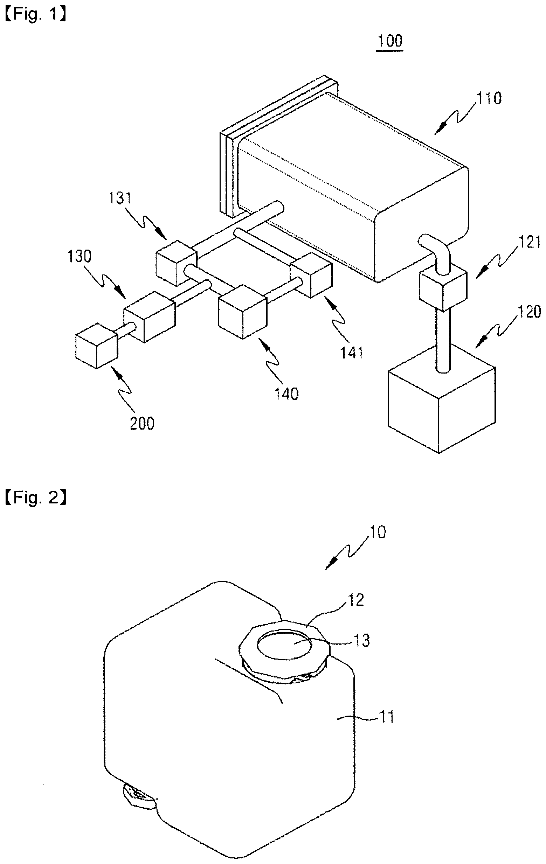

FIG. 1 is a schematic perspective view illustrating a configuration of a sterilizer 100 to which a hydrogen peroxide supply apparatus 200 according to the present invention is applied, and FIG. 2 is a perspective view illustrating a hydrogen peroxide bottle 10 mounted on the hydrogen peroxide supply apparatus 200 of FIG. 1.

At this time, in the embodiment of the present invention, since hydrogen peroxide solution is used as the disinfectant solution, the disinfectant container is referred to as a hydrogen peroxide bottle 10, and the disinfectant solution is not limited to hydrogen peroxide solution in the present invention.

Therefore, when the hydrogen peroxide tank 10 is filled with another disinfectant solution instead of the hydrogen peroxide, the hydrogen peroxide bottle 10 may be referred to as a general disinfectant container.

Referring to FIG. 1, the sterilizer 100 to which the hydrogen peroxide supply apparatus 200 according to the present invention is applied is an apparatus which drains hydrogen peroxide of a liquid state from the hydrogen peroxide bottle 10 housed in the hydrogen peroxide supply apparatus 200 and vaporizes it, and then sterilizes and disinfects the object to be sterilized.

The sterilizer 100 includes a sterilization chamber 110 capable of containing sterilized materials such as medical devices or surgical tools to be sterilized, and a vacuum pump 120 connected to one side of the sterilization chamber 110, and the vacuum pump 120 is configured to extract a gas inside the sterilization chamber 110 to form a vacuum state. At this time, a vacuum valve 121 capable of controlling the operation of the vacuum pump 120 is connected between the sterilization chamber 110 and the vacuum pump 120.

Subsequently, the sterilizer 100 includes a vaporizer 130 (or may be referred to as an evaporator) which is connected to the other side of the sterilization chamber 110 to supply hydrogen peroxide vapour to the sterilization chamber 110, and at this time, a vaporization valve 131 may be included between the sterilization chamber 110 and the vaporizer 130.

Meanwhile, a hydrogen peroxide supply apparatus 200 for supplying hydrogen peroxide according to the present invention may be connected to the vaporizer 130.

Further, one side of the sterilizer 100 is connected to the vaporizer 130, the other side thereof is connected to the sterilization chamber 110, and the sterilizer 100 includes a collector 140 (or may be referred to as a collecting vaporizer) for concentrating hydrogen peroxide supplied to the vaporizer 130.

At this time, a vaporization valve 131 is provided between the sterilization chamber 110 and the collector 140, and a collection valve 141 may be provided between the sterilization chamber 110 and the collector 140.

Meanwhile, although not illustrated in the drawing, temperature control means for controlling the temperatures of the sterilization chamber 110, the vaporizer 130 and the collector 140 may be included, and the temperature control means may be a heater, and since this is a matter obvious in the art, a specific description thereof will not be provided.

Meanwhile, the hydrogen peroxide bottle 10 applied to the hydrogen peroxide supply apparatus 200 of the present invention with reference to FIG. 2 is not transient but is a disinfectant container filled with a large volume of hydrogen peroxide. In general, since the containers filled with high-concentration hydrogen peroxide for sterilization and disinfection which are distributed in the market have high vaporability, if there is a severe vaporization during the distribution process, there is a danger of explosion due to the bulging of container.

Therefore, as illustrated in FIG. 2, the large-capacity hydrogen peroxide bottle 10 mounted on the hydrogen peroxide supply apparatus 200 of the present invention is desirably distributed, by being sealed with a lid 12 including a porous film 13 through which hydrogen peroxide solution of a liquid state cannot pass and only hydrogen peroxide vapour vaporized in a gaseous state can pass, together with the container 11 storing hydrogen peroxide solution.

Next, the hydrogen peroxide supply apparatus 200 of the present invention which is connected to the vaporizer 130 of the sterilizer 100 to supply hydrogen peroxide solution will be described.

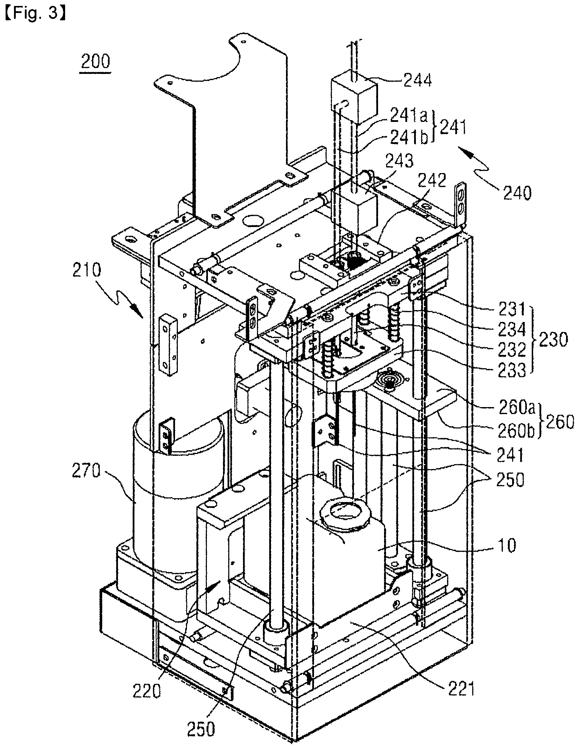

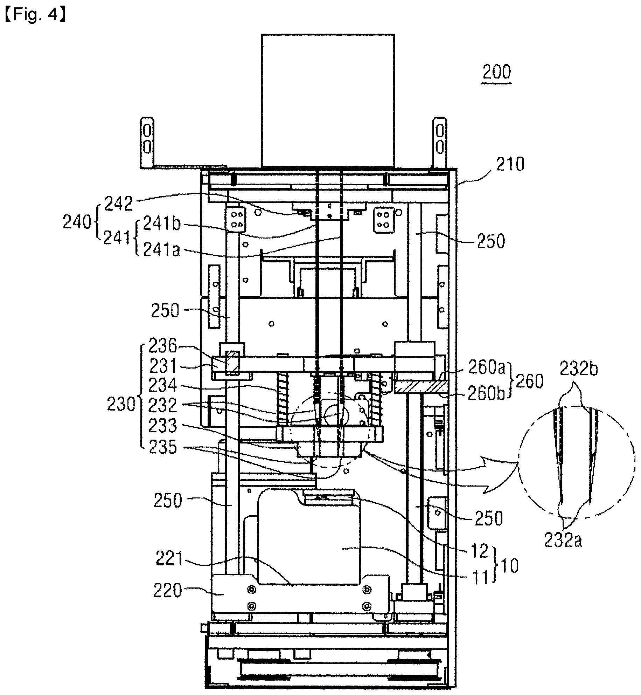

FIG. 3 is a perspective view illustrating the hydrogen peroxide supply apparatus of the present invention, and FIG. 4 is a cross-sectional view thereof.

Referring to FIGS. 3 and 4, the hydrogen peroxide supply apparatus 200 according to the present invention basically includes a disinfectant lifting and lowering unit 220, a stinger unit 230 and a drain unit 240, and may be included inside the housing 210.

Further, the hydrogen peroxide supply apparatus 200 may further include a driving means 270 and a transport guide 250. The driving means 270 may be a reversible motor which rotates in normal and reverse directions. The lifting and lowering motions of the disinfectant lifting and lowering unit 220 may be performed along the transport guide 250 by the operation of the reversible motor.

More specifically, the transport guide 250 receives the transmission force of the driving means 270 by another power transmission device (not illustrated) to allow the transmission of the disinfectant lifting and lowering unit 220 and the stinger unit 230. A plurality of transport guides 250 may be provided inside the housing 210 in the form of a rod, a rail or a bar.

Meanwhile, when the sterilization lifting and lowering unit 220 and the stinger unit 230 moves up and down, while sliding on the transport guide 250, a transport locking unit 260 for performing the role of locking jaw at the time of lifting and lowering motion of the sterilization lifting and lowering unit 220 may be provided on one side in the housing 210.

Subsequently, the disinfectant lifting and lowering unit 220 includes a storage unit 221 for mounting the hydrogen peroxide bottle 10, as a device for mounting and transporting the hydrogen peroxide bottle 10 filled with hydrogen peroxide solution as disinfectant to the hydrogen peroxide supply apparatus 200. The hydrogen peroxide bottle 10 with the hydrogen peroxide solution exhausted may be exchanged through the storage unit 221.

Further, the disinfectant lifting and lowering unit 220 is provided slidably along the transport guide 250 fixed in the housing 210, and the lifting and lowering motion is performed by the operation of the driving means.

For example, in the initial state before the hydrogen peroxide bottle 10 is housed in the storage unit 221, the disinfectant lifting and lowering unit 220 is located at the bottom of the housing 210, and after the hydrogen peroxide bottle 10 is housed in the storage unit 221, the disinfectant lifting and lowering unit 220 moves up and down along the transport guide 250 by the operation of the driving means 270.

Next, the initially sealed state of the hydrogen peroxide bottle 10 lifted and lowered by the disinfectant lifting and lowering unit 220 may be released by the stinger unit 230.

Referring to FIGS. 3 and 4, the stinger unit 230 may further include, as a device for piercing and opening the lid 12 of the initially sealed hydrogen peroxide bottle 10, a transport unit 231 which moves up and down along the transport guide 250; a stinger 232 which is fixed to the bottom of the transport unit 231 and pieces and opens the lid of the hydrogen peroxide bottle 10; a fixing unit 233 to which the lid 123 of the hydrogen peroxide bottle 10 is inserted and fixed; and an elastic unit 234 which connects the transport unit 231 and the fixing unit 233. The fixing unit 233 may further include a through-hole 235 through which the stinger 232 is guided.

At this time, the stinger 232 may be configured to include a tip portion 232a having a pointed shape and a tubular body portion 232b having a hollow inner diameter so that the stinger can penetrate the lid 12 of the hydrogen peroxide bottle 10.

In addition, in the embodiment of the present invention, the shape of the tip portion 232a is illustrated to form a pointed tip portion at an oblique slope. However, in addition to this, tip portion 232a may be manufactured in any shape that can penetrate the lid of the hydrogen peroxide bottle 10, such as a V-shape.

Meanwhile, referring to FIG. 4, as described above in the description of the stinger unit 230, the drain pipe 241 is provided in a state of being partly inserted into the inner diameter of the stinger body portion 232b, and the inner diameter of the stinger 232 is formed to be larger than the outer diameter of the drain pipe 241 so that a drain pipe 241 to be described later can be inserted.

Subsequently, the drain unit 240 is configured to include a drain pipe 241 which drains the hydrogen peroxide from the hydrogen peroxide bottle 10 to the vaporizer 130, and a support means 242 for fixing the drain pipe 241 to the housing 210.

The drain pipe 241 is provided in a tubular shape such as a straw so that the hydrogen peroxide solution can be drained from the hydrogen peroxide bottle 10 and supplied to the vaporizer 130, and the drain pipe 241 is fixed to upper part of the housing 210 by the support means 242.

Meanwhile, since it is very important that a fixed amount of aqueous hydrogen peroxide solution is supplied to the vaporizer 130 of the sterilizer 100 due to the characteristics of the sterilizer 100 which uses the aqueous hydrogen peroxide solution as a disinfectant, it is necessary to precisely control the amount of the aqueous hydrogen peroxide solution to be supplied.

Meanwhile, when only the supply drain pipe that supplies the hydrogen peroxide solution to the vaporizer 130 is provided alone, air bubbles are generated in the supply drain pipe after performing the sterilization process or the hydrogen peroxide may remain after the sterilization process, there is a problem that it is difficult to precisely control the amount of hydrogen peroxide when the new sterilization process is repeated.

Therefore, a plurality of drain pipes 241 of the present invention may be provided instead of a single piece, and in the embodiment of the present invention, the drain pipe 241 may include a first drain pipe 241a and a second drain pipe 241b.

At this time, when the first drain pipe 241a is a supply drain pipe, the second drain pipe 241b may be a discharge drain pipe, and when the first drain pipe 241a is a discharge drain pipe, the second drain pipe 241b may be a supply drain pipe.

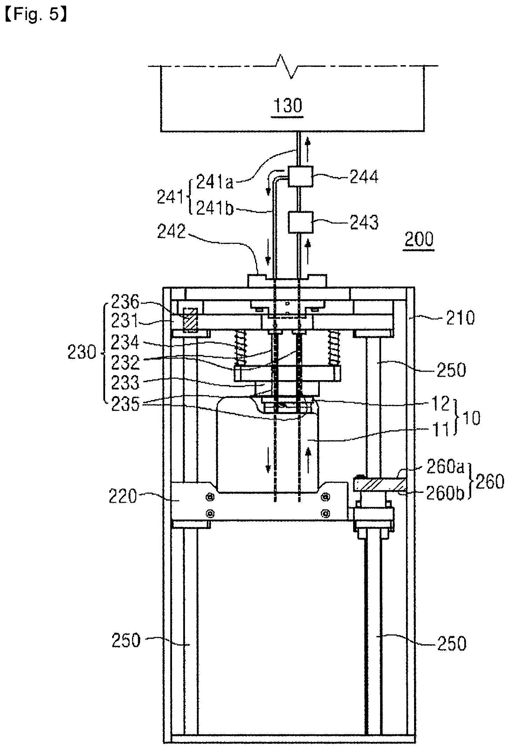

FIG. 5 is a view illustrating a state in which the hydrogen peroxide supply apparatus 200 of the present invention is connected to the vaporizer 130 of the sterilizer 100.

Referring to FIG. 5, the drain pipe 241 includes a first drain pipe 241a which supplies the disinfectant drained from the disinfectant container 10 to the sterilizer 100 241a, and a second drain pipe 241 which is diverged from a point of the first drain pipe 241a at a position close to the sterilizer 100 to re-discharge or circulate the residue inside the first drain pipe 241a to the disinfectant container 10.

At this time, the first drain pipe 241a and the second drain pipe 241b are fixed and supported by the support means 242 provided on the upper part of the housing 210, and all one end portions of the first drain pipe 241a and the second drain pipe 241b can be inserted into the hydrogen peroxide bottle 10.

Meanwhile, the other end portion of the first drain pipe 241a functions as a supply drain pipe which extends to the vaporizer 130 of the sterilizer 100 to supply the hydrogen peroxide solution into the vaporizer 130. At this time, a supply pump 243 is further included on the pipeline of the first drain pipe 241a to drain the hydrogen peroxide solution as the disinfectant and supply it to the vaporizer 130. As the supply pump 243, a small amount precision pump or the like generally used such as a tubing pump, a gear pump, and a piston pump may be applied.

Further, the other end portion of the second drain pipe 241b is not directly connected to the vaporizer 130, and one point of the first drain pipe 241a close to the vaporizer 130 is connected to the pipe. That is, this is similar to a configuration in which the second drain pipe 241b is diverged from any point of the first drain pipe 241a and inserted into the hydrogen peroxide bottle 10. A three-way valve 244 may be provided at the connection point. Since the first drain pipe 241a is diverged by the three-way valve 244, selective flow passage switching is enabled such that hydrogen peroxide is supplied to the vaporizer 130 or the second drain pipe 241b.

At this time, the second drain pipe 241b performs a discharge drain pipe which discharges or circulates air bubbles or hydrogen peroxide solution remaining in the first drain pipe 241a to the hydrogen peroxide bottle 10 of the hydrogen peroxide supply apparatus 200 again.

That is, the first drain pipe 241a may directly supply the disinfectant sucked from the disinfectant container 10 to the vaporizer 130, or switch the flow of the flow passage by the three-way valve 244, send the disinfectant to the second drain pipe 241b again and discharge the disinfectant to the disinfectant container 10 again.

Normally, the process of sending the disinfectant sucked from the first drain pipe 241a to the second drain pipe 241b and discharging the disinfectant to the disinfectant container 10 is desirably performed immediately before supplying the hydrogen peroxide to the vaporizer 130 every time the sterilizing apparatus 100 is operated. The reason is that, before supplying the hydrogen peroxide to the vaporizer 130, through the process of discharging the hydrogen peroxide sucked from the first drain pipe 241a to the second drain pipe 241b, a work of removing the air bubbles that may remain in the pipeline after the disinfection process is finished beforehand to fill the pipeline with hydrogen peroxide solution is first performed.

After the discharge process is completed, hydrogen peroxide needs to be supplied to the vaporizer 130 for operation of the sterilizer 100. To this end, the second drain pipe 241b as the discharge drain pipe is blocked by the operation of the three-way valve 244, and the pipeline of the first drain pipe 241a as the supply drain pipe is immediately connected to the vaporizer 130 to supply the hydrogen peroxide to the vaporizer 130 so that disinfection can be performed.

Therefore, when the sterilization process is performed in the future after the sterilization process is finished by supplying hydrogen peroxide to the vaporizer 130 through the first drain pipe 241a, through a process in which air bubbles or the remaining hydrogen peroxide interfering with a constant supply of the hydrogen peroxide are sucked to the second drain pipe 241b and are discharged or circulated through the hydrogen peroxide bottle 10 by controlling the 3-way valve 244, even if the sterilization work is performed several times, the constant amount of hydrogen peroxide can be continuously supplied.

Also, the operation of the supply drain pipe or the discharge drain pipe can be performed through another control device such as a sensor.

Next, the operation principle according to the configuration of the hydrogen peroxide supply apparatus 200 of the present invention will be described.

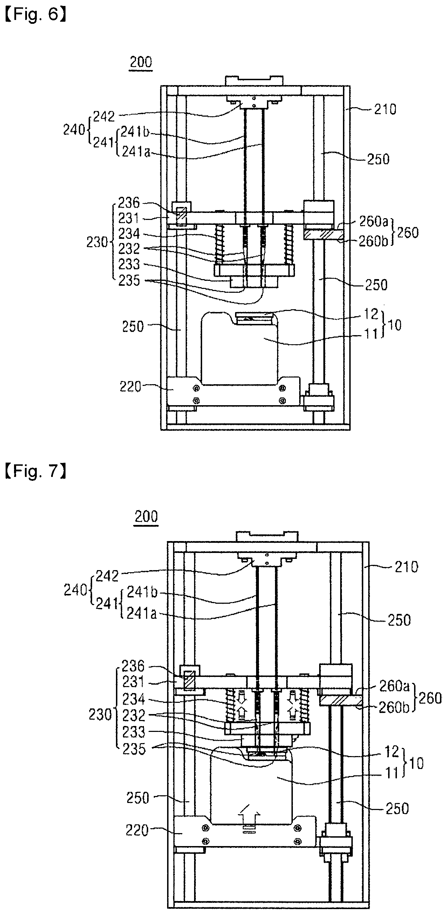

FIGS. 6 to 10 are schematic cross-sectional views illustrating the operation procedure of the hydrogen peroxide supply apparatus 200 according to the present invention, and illustrating an operation principle in which the disinfectant lifting and lowering unit 220 and the stinger unit 230 move up and down in the transport guide 250 and the operation principle in which the hydrogen peroxide solution is drained from the drain unit 240.

First, FIG. 6 illustrates an initial state before operation of the hydrogen peroxide transport device 200 of the present invention, the sterilization lifting and lowering unit 220 of the initial state is mounted on the transport guide 250 to move up and down and is located at the bottom of the housing 210. At this time, the hydrogen peroxide bottle 10 is seated on the storage unit 221 of the sterilization lifting and lowering unit 220.

At this time, the hydrogen peroxide bottle 10 is seated on the storage unit 221 of the hydrogen peroxide supply apparatus 200 in a state in which the lid 12 is sealed with the hydrogen peroxide solution as disinfectant filled therein.

Next, the stinger unit 230 is also provided to move up and down along the transport guide 250, and in the initial state before the operation of the hydrogen peroxide supply apparatus 200, the transport unit 231 of the stinger unit 230 is located in the state of being fixed to the transport locking unit 260 provided on the intermediate side portion of the housing 210, more specifically, the upper surface 260a of the transport locking unit.

In this way, the disinfectant lifting and lowering unit 220 and the stinger unit 230 are provided to move up and down along the transport guide 250 in the housing 210. Meanwhile, the drain unit 240 is provided in a state of being fixed to the upper part of the housing 210 without a change in the position. More specifically, the drain pipe 241 is provided to protrude downward from the upper part of the housing 210 by the support means 242.

Subsequently, as illustrated in FIG. 7, the driving means 270 is operated to lift and lower the disinfectant lifting and lowering unit 220 which houses the hydrogen peroxide bottle 10.

At this time, while the sterilization lifting and lowering unit 220 moves up and down along the transport guide 250, the hydrogen peroxide bottle 10 housed in the sterilization lifting and lowering unit 220 becomes gradually close to the stinger unit 230 which has been stopped on the upper surface 260a of the transport locking unit in the initial state, and at the moment when the lid 12 located at the upper part of the hydrogen peroxide bottle 10 being lifted and lowered continues to move up and down to come into contact with the fixing unit 233 of the stinger unit 230, the lid 12 is fixed while being inserted into the fixing unit 233.

In this way, at the moment when the lid 12 comes into contact with the fixing unit 233, in a state in which the transport unit 231 which has been stopped from the initial state receives the force of gravity and is stopped as it is, the lid 12 being lifted and lowered pushes the fixing unit 233 upward, while applying force to the bottom of the abutting fixing unit 233.

At this time, the transport unit 231 can be in a stopped state without being lifted upward until piercing the sealed lid 12 due to action of gravity caused by its own weight of the transport unit 231. However, it is possible to include another fixing means 236 so that the transport unit 231 can be stopped until the transport unit 231 pierces the sealed lid for more secure opening of the lid.

The fixing means 236 may be a braking device such as a brace or an electronic brake, or a fixing means made of a magnetic material. In the embodiment of the present invention, the fixing means 236 uses an electromagnet (see FIG. 5).

Therefore, a compression force acts on the elastic portion 234 which connects the transport unit 231 and the fixing unit 233, and the interval between the transport unit 231 and the fixing unit 233 becomes narrower. At this time, the stinger 232 attached to the lower part of the transport unit 231 passes through the through-hole 235 provided in the fixing unit 233 to protrude to the lower part of the fixing unit 233, and pierces and opens the sealed lid 12 of the hydrogen peroxide bottle 10 which is in contact with the bottom of the fixing unit 233.

In this way, when the elastic force acts on the elastic portion 234 to narrow the interval between the lifting and lowering unit 231 and the fixing unit 233, while the stinger 232 protrudes through the through-hole 235 of the fixing unit 233, the lid 12 of the sealed hydrogen peroxide bottle 10 is pierced and opened, and a drain pipe 241 to described later is prepared so as to be inserted.

Meanwhile, when an elastic restoring force acts on the elastic portion 234 to keep the interval between the lifting and lowering unit 231 and the fixing unit 233 away from each other, the stinger 232 protruded through the through-hole 235 of the fixing unit 233 is located again in the upward direction and is extracted from the lid 12 of the hydrogen peroxide bottle 10.

For reference, in the embodiment of the present invention, the operation process of the fixing means 236 using the electromagnet is as follows.

When the disinfectant lifting and lowering unit 220 moves up and down to reach the position of the stinger unit 230 as illustrated in FIG. 7, in the stinger unit 230, only the fixing unit 233 moves upward by deformation of the elastic unit 234, and at this time, the transport unit 231 is fixed in the stopped state, in a state in which the magnetic field of the electromagnet 236 operates at this time. Therefore, the stinger 232 passes through the through-hole 235 of the fixing unit 233 and penetrates the sealed lid 12.

Next, when releasing magnetic field of the electromagnet after piercing the lid 12, the supply drain pipe 231a and the discharge drain pipe 231b located inside the through-hole 235 are inserted into the disinfectant container 10, the disinfectant lifting and lowering unit 220 moves upward to a certain position to lift the disinfectant container 10 together.

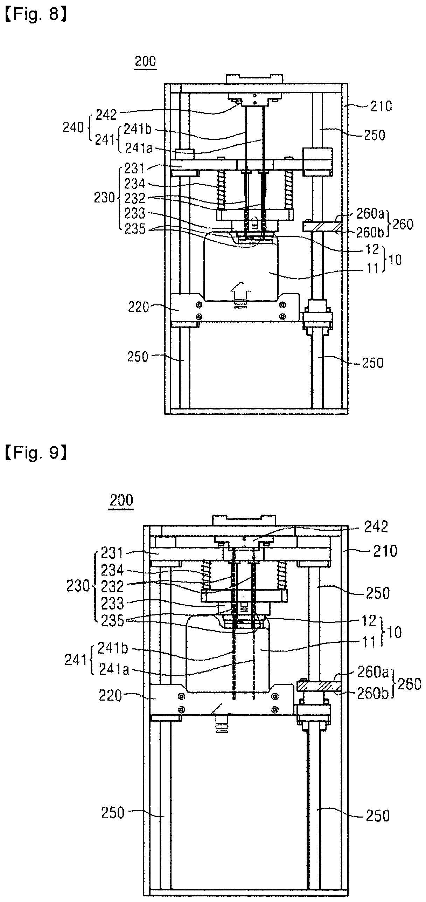

Next, as illustrated in FIG. 8, a drain pipe 241 is inserted into the hydrogen peroxide bottle 10 in which the lid 12 has been opened, such that the hydrogen peroxide solution can be sucked, drained and supplied to the vaporizer 130 of the sterilizer 100.

As illustrated in FIG. 8, the disinfectant lifting and lowering unit 220 continuously moves up and down inside the housing 210 along the transport guide 250, and the force for pushing up the fixing unit 233 of the stinger unit 230 by the lid 12 of the hydrogen peroxide bottle 10 becomes larger, and the pushing-up force is also to transmitted to the transport unit 231 stopped in a state of being connected with the fixing unit 233 by the elastic unit 234. As a result, the whole stinger unit 230 will move up and down along the transport guide 250.

At this time, the lifting and lowering movement of the stinger unit 230 can be performed only by the force in which the hydrogen peroxide bottle 10 housed in the disinfectant lifting and lowering device 220 abuts against and pushes up the fixing unit 233 of the stinger unit 230, while moving upward and downward, without another power unit.

Meanwhile, when the stinger unit 232 continues to rise in a state in which the tip portion 232a of the stinger 232 is inserted into the lid 12 of the hydrogen peroxide bottle by piercing the opening, the hydrogen peroxide bottle 10 reaches the position of the lower end suction port of the drain pipe 241. In this way, the drain pipe 241 is finally inserted, while the hydrogen peroxide bottle 10 continuously moves up and down.

Meanwhile, as illustrated in FIG. 8, since a part of the lower end of the drain pipe 241 is inserted into the inner diameter of the body portion 232b of the sting unit 232 from the initial state, the inner diameter of the body portion 232b of the stinger unit 232 serves as a guide, and with the drain pipe 241 inserted, the stinger unit 232 moves upward and downward.

Therefore, after the hydrogen peroxide bottle 10 is lifted and lowered, and as illustrated in FIG. 7, the stinger unit 232 pierces and opens the sealed lid 12 of the hydrogen peroxide bottle 10, as in FIG. 8 as the next step, when the hydrogen peroxide bottle 10 continuously moves up and down to reach the position of the drain pipe 241, an suction port of the lower end of the drain pipe 241 is naturally guided while being inserted in the inner diameter of the body portion of the stinger unit 232 and can be naturally inserted into the lid 12 of the hydrogen peroxide bottle 10.

At the same time, the pushing-up force of the hydrogen peroxide bottle 10 is also transmitted to the stopped transport unit 231 by the elastic unit 234, and while the transport unit 231 moves up and down along the transport guide 250, the elastic force of the elastic portion 234 is restored. Accordingly, the stinger 232, which has protruded to the lower part of the fixing unit 233 through the through-hole 235 provided in the fixing unit 233, rises again to the upper part of the fixing unit 233 and is located at an original position.

Subsequently, as illustrated in FIG. 9, the disinfectant lifting and lowering unit 220 which has been continuously moved up and down by the driving means 270 is locked by the lower surface 260b of the transport locking unit 260, and is stopped without further lifting and lowering motion.

Further, since the suction port of the lower end of the drain pipe 241 reaches the bottom of the hydrogen peroxide cylinder 10, the hydrogen peroxide stored in the hydrogen peroxide bottle 10 can be used until it is exhausted, without replacing the hydrogen peroxide bottle 10.

Meanwhile, when the sterilizer 100 is operated, the vaporizer 130 is evacuated, and the hydrogen peroxide of the value set to an amount suitable for single sterilization disinfection is drained into the interior of the vaporizer 130 which maintains the state of appropriate temperature and pressure.

At this time, the hydrogen peroxide is drawn from the hydrogen peroxide bottle 10 housed in the hydrogen peroxide supply apparatus 200 of the present invention through the drain pipe 241, and is drained into the interior of the vaporizer 130 of the sterilizer 100 through another nozzle connected to the drain pipe 241.

Meanwhile, before supplying the hydrogen peroxide to the vaporizer 130 as described above, the work of discharging the hydrogen peroxide to the discharge drain pipe 241b after sucked into the supply drain pipe 241a is first performed, and a work of filling the pipeline with hydrogen peroxide without air bubbles is first performed. If such a process is controlled by the 3-way valve 244 and the supply pump 243, an appropriate amount of hydrogen peroxide can be precisely supplied to the vaporizer 130.

Next, the sterilizer 100 vaporizes the appropriate amount of hydrogen peroxide solution of the drain into the vaporizer 130 to form a highly concentrated hydrogen peroxide solution. Subsequently, the highly concentrated hydrogen peroxide solution is subjected to the discharge and concentration steps in the collector 140 of the sterilizer 100, and vapour of the concentrated hydrogen peroxide solution is introduced into the sterilization chamber 110 to sterilize the processed material.

Next, the hydrogen peroxide bottle 10 in which hydrogen peroxide is exhausted through the sterilization process several times can be used by being exchanged for a new hydrogen peroxide bottle 10 again.

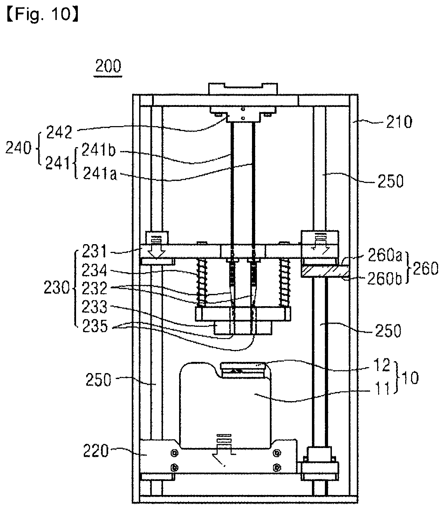

To this end, as illustrated in FIG. 10, the reversing motor as the driving means 270 is reversely rotated so that the disinfectant lifting and lowering unit 220 lifted and lowered to the lower surface 260b of the transport locking unit 210 is transported to the bottom of the housing 210 of the initial state again.

For reference, during transportation of the disinfectant lifting and lowering unit 220 to the bottom of the housing 210, the suction port of the drain pipe 241 can be exposed over the remaining liquid surface. At this time, after the process of driving the pump 243 to discharge all the liquid in the pipeline to the hydrogen peroxide bottle 10, it is desirable to transport the hydrogen peroxide bottle 10 to the bottom of the housing 210.

Next, it is possible to release the exhausted hydrogen peroxide bottle 10 from the storage unit 221 of the sterilization lifting and lowering unit 220 which has been lowered to the bottom of the housing 210 along the transport guide 250. Meanwhile, the hydrogen peroxide bottle 10 exhausted while repeating the procedures of FIGS. 6 to 10 can be used by being replaced with a new hydrogen peroxide bottle 10'.

### INDUSTRIAL APPLICABILITY

According to the present invention as described above, since the hydrogen peroxide supply apparatus 200 of the present invention is used while mounting the high-capacity hydrogen peroxide bottle 10, the supply of hydrogen peroxide can be performed many times. Therefore, there is an effect that the operation of the sterilizer is convenient without frequent exchange of the container for hydrogen peroxide as disinfectant, and the sterilization is quickly performed.

In addition, the present invention has an effect of quantitatively supplying a small amount of hydrogen peroxide liquid to the sterilizer, while having a simple apparatus structure.

Embodiments of the present invention have been described with reference to the accompanying drawings above. However, those having ordinary knowledge in the technical field to which the present invention pertains will understand that the present invention can be embodied in other specific forms without changing the technical idea or essential features thereof. It is therefore to be understood that the above-described embodiments are illustrative in all aspects and not restrictive.

### Reference Numerals

10 hydrogen peroxide bottle
100 sterilizer
110 sterilization chamber
120 vacuum pump
121 vacuum valve
130 vaporizer
131 vaporization valve
140 collector
141 collection valve
200 hydrogen peroxide supply apparatus
210 housing
220 disinfectant lifting and lowering unit
221 storage unit
230 stinger unit
231 transport
232 stinger
233 fixing unit
234 elastic unit
235 through-hole
240 drain unit
241 drain pipe
242 support means
243 supply pump
244 three-way valve
250 transport guide
260 transport locking unit
270 driving means

## Claims

1. A hydrogen peroxide supply device comprising:
a disinfectant lifting and lowering unit which transports a disinfectant container;
a stinger unit which opens the disinfectant container;
a drain unit which drains disinfectant from the disinfectant container; and
a transport guide which transports the disinfectant lifting and lowering unit and the stinger unit.

2. The hydrogen peroxide supply device of claim 1, wherein the stinger unit further comprises:
a transport unit which moves up and down along the transport guide;
a stinger fixed to the bottom of the transport unit to open the disinfectant container;
a fixing unit to which a lid of the disinfectant container is inserted and fixed; and
an elastic unit which connects the transport and the fixing unit,
wherein the fixing unit further includes a through-hole through which the stinger is guided.

3. The hydrogen peroxide supply device of claim 2, wherein the stinger comprises:
a tip portion which opens the lid of the disinfectant container; and
a tubular body portion.

4. The hydrogen peroxide supply device of claim 1, wherein the drain unit comprises:
a drain pipe which drains the disinfectant; and
support means for fixing the drain pipe to the housing.

5. The hydrogen peroxide supply device of claim 3 or 4, wherein the drain pipe is provided in a state in which a part thereof is inserted into an inner diameter of the stinger body portion, and
the inner diameter of the stinger body portion is formed to be larger than an outer diameter of the drain pipe.

6. The hydrogen peroxide supply device of claim 4, wherein the drain pipe comprises:
a first drain pipe which supplies the disinfectant drained from the disinfectant container to the sterilizer; and
a second drain pipe which is diverged from a point of the first drain pipe at a position close to the sterilizer to re-discharge or circulate the residue inside the first drain pipe to the disinfectant container.
